# EUROPEAN PATENT APPLICATION

(11) **EP 2 963 052 A2**
(43) Date of publication of application: **06.01.2016**
(21) Application number: 14734215.8
(22) Date of filing: 26.02.2014
(51) Int. Cl.: C07K 5/072, C07K 5/083, C07K 5/103, A61K 38/00

(54) **PEPTIDES WITH CYTOPROTECTIVE ACTIVITY**

(30) Priority: 28.02.2013 EA 201300197
(71) Applicant: Zamerton Holdings Limited, 6018 Larnaka (CY)
(72) Inventor: OVCHINNIKOV, Mihail Vladimirovich, 115162 Moscow (RU); CHERTORIZHSKIY, Evgeniy Aleksandrovich, 249031 Obninsk Kaluzhskaya region (RU); BELY, Petr Aleksandrovich, 127055 Moscow (RU)
(74) Representative: Icosa
(86) International application number: PCT/IB2014/000456
(87) International publication number: WO 2014/162190

(57) **Abstract**

This invention is related to medicine and concerns peptides with cytoprotective activity selected from a group that includes Ac-Asp-Glu-OH, H-Asp-Glu-OCH₃, H-Asp-Glu-NH - CH₂-CH₃, H-Val-Asp-Glu-OH, H-Leu-Asp- Glu-OH and H-Ala-Asp-Glu-Arg-OH, Ala-Asp-Glu, Lys-Asp-Glu and Asp-Glu-Gly, with the peptides remaining chemically stable in storage. Also proposed is a pharmaceutical composition containing effective quantities of peptides selected from a group that includes Ac-Asp-Glu-OH, H-Asp-Glu-OCH₃, H-Asp-Glu-NH - CH₂-CH₃, H-Val-Asp-Glu-OH, H-Leu-Asp- Glu-OH and H-Ala-Asp-Glu-Arg-OH, Ala-Asp-Glu, Lys-Asp-Glu or Asp-Glu-Gly.

The pronounced cytoprotective effect makes it possible to use the pharmaceutical composition in the food, cosmetological and pharmaceutical industries.

## Description

### FIELD OF THE INVENTION

This invention relates to medicine and concerns biologically active peptides with cytoprotective effect, and a pharmaceutical composition based on these peptides.

### DESCRIPTION OF THE RELATED ART

There are a number of known short peptides whose structure contains derivatives of the peptide Y-L-Glu-L-Asp-X and that exhibit the qualities of intercellular mediators [Patents RU No. 2177802; 2161501; 2301074; 2304444; 2155063]. Their shortcoming is chemical instability and propensity for spontaneous regrouping. If X is a residue of glycine, this residue may spontaneously migrate from α-carboxyl of aspartic acid to β-carboxyl. Although this transformation may occur to any amino acid the speed of the reaction depends heavily on steric hindrances [Pharmaceutical Research, Vol. 11, N 5, 1994, p. 751-758; Pharmaceutical Research, Vol.7, No. 8, 1990, p. 787-793]. If X is a residue of proline or any other organic compound with a secondary amine group, the bound between these residues is spontaneously broken [Synthetic peptides, Sec. ed., Gregory A. Grant, Oxford Univ. Press 2002, p. 283]. It should be noted that these transformations lead to partial racemization of the residue of aspartic acid.

### SUMMARY

The authors of this invention are the first to propose peptides having cytoprotective activity selected from a group that includes Ac-Asp-Glu-OH (SEQ ID NO: 1), H-Asp-Glu-OCH₃ (SEQ ID NO: 2), H-Asp-Glu-NH-CH₂-CH₃ (SEQ ID NO: 3), H-Val-Asp-Glu-OH (SEQ ID NO: 4), H-Leu-Asp-Glu-OH (SEQ ID NO: 5) and H-Ala-Asp-Glu-Arg-OH (SEQ ID NO: 6). The peptides proposed in this invention have a pronounced cytoprotective activity and are chemically stable upon storage.

Another aspect of this invention is a pharmaceutical composition having a cytoprotective activity and contains an effective quantity of peptides selected from a group that includes Ac-Asp-Glu-OH (SEQ ID NO: 1), H-Asp-Glu-OCH₃ (SEQ ID NO: 2), H-Asp-Glu-NH-CH₂-CH₃ (SEQ ID NO: 3), H-Val-Asp-Glu-OH (SEQ ID NO: 4), H-Leu-Asp-Glu-OH (SEQ ID NO: 5) or H-Ala-Asp-Glu-Arg-OH (SEQ ID NO: 6).

### BRIEF DESCRIPTION OF THE FIGURES

**Fig. 1****.** Number of cells in a vial and the ratio of dead PI+ cells after incubation with the peptide H-Asp-Glu-NH - CH₂-CH₃ (DENHEt) (SEQ ID NO: 3).
**Fig. 2****.** Number of cells in a vial and the ratio of dead PI+ cells after incubation with the peptide Ac-Asp-Glu-OH (AcDE) (SEQ ID NO: 1).
**Fig. 3****.** Number of cells in a vial and the ratio of dead PI+ cells after incubation with the peptide H-Asp-Glu-OCH3 (DEOMe)
**Fig. 4****.** Number of cells in a vial and the ratio of dead PI+ cells after incubation with the peptide H-Val-Asp-Glu-OH (VDE).
**Fig. 5****.** Number of cells in a vial and the ratio of dead PI+ cells after incubation with the peptide H-Leu-Asp-Glu-OH (LDE) (SEQ ID NO: 5).
**Fig. 6****.** Number of cells in a vial and the ratio of dead PI+ cells after incubation with the peptide H-Ala-Asp-Glu-Arg-OH (ADER) (SEQ ID NO: 6).
**Fig. 7****.** Rate of apoptotic cell death of HeLa S3 cells, assessed as a ratio of cells with hypodiploid content of DNA, 24 hours after incubation with the peptide H-Asp-Glu-NH - CH₂-CH₃ (DENHEt) (SEQ ID NO: 3).
**Fig. 8****.** Rate of apoptotic cell death of HeLa S3 cells , assessed as a ratio of cells with hypodiploid content of DNA, 24 hours after incubation with the peptide Ac-Asp-Glu-OH (AcDE) (SEQ ID NO: 1).
**Fig. 9****.** Rate of apoptotic cell death of HeLa S3 cells, assessed as a ratio of cells with hypodiploid content of DNA, 24 hours after incubation with the peptide H-Asp-Glu-OCH3 (DEOMe).
**Fig. 10****.** Rate of apoptotic cell death of HeLa S3 cells, assessed as a ratio of cells with hypodiploid content of DNA, 24 hours after incubation with the peptide VDE (SEQ ID NO: 4).
**Fig. 11****.** Rate of apoptotic cell death of HeLa S3 cells, assessed as a ratio of cells with hypodiploid content of DNA, 24 hours after incubation with the peptide LDE.
**Fig. 12****.** Rate of apoptotic cell death of HeLa S3 cells, assessed as a ratio of cells with hypodiploid content of DNA, 24 hours after incubation with the peptide ADER (SEQ ID NO: 6)
**Fig. 13****.** Protective effect of the peptide H-Asp-Glu-NH - CH₂-CH₃ (DENHEt) (SEQ ID NO: 3) on HeLa cells in the presence of camptothecin.
**Fig. 14****.** Protective effect of the peptide H-Asp-Glu-NH - CH₂-CH₃ (DENHEt) (SEQ ID NO: 3) on NIH3T3 cells in the presence of camptothecin.
**Fig. 15****.** Protective effect of the peptide Ac-Asp-Glu-OH (AcDE) (SEQ ID NO: 1) on NIH3T3 cells in the presence of camptothecin.
**Fig. 16****.** Protective effect of the peptide Ac-Asp-Glu-OH (AcDE) (SEQ ID NO: 1) on HeLa cells in the presence of camptothecin.
**Fig. 17****.** Protective effect of the peptide H-Asp-Glu-OCH3 (DEOMe) (SEQ ID NO: 2) on HeLa cells in the presence of camptothecin.
**Fig. 18****.** Protective effect of the peptide H-Asp-Glu-OCH3 (DEOMe) (SEQ ID NO: 2) on NIH3T3 cells in the presence of camptothecin.
**Fig. 19****.** Protective effect of the peptide H-Val-Asp-Glu-OH (VDE) (SEQ ID NO: 4) on NIH3T3 cells in the presence of camptothecin.
**Fig. 20****.** Protective effect of the peptide H-Val-Asp-Glu-OH (VDE) (SEQ ID NO: 4) on HeLa cells in the presence of camptothecin.
**Fig. 21****.** Protective effect of the peptide H-Leu-Asp-Glu-OH (LDE) (SEQ ID NO: 5) on HeLa cells in the presence of camptothecin.
**Fig. 22****.** Protective effect of the peptide H-Leu-Asp-Glu-OH (LDE) (SEQ ID NO: 5) on NIH3T3 cells in the presence of camptothecin.
**Fig. 23****.** Protective effect of the peptide H-Ala-Asp-Glu-Arg-OH (ADER) (SEQ ID NO: 6) on NIH3T3 cells in the presence of camptothecin.
**Fig. 24****.** Protective effect of the peptide H-Ala-Asp-Glu-Arg-OH (ADER) (SEQ ID NO: 6) on HeLa cells in the presence of camptothecin.

### DETAILED DESCRIPTION

The present invention first provides biologically active peptides having a cytoprotective activity selected from a group that includes Ac-Asp-Glu-OH (SEQ ID NO: 1), H-Asp-Glu-OCH₃ (SEQ ID NO: 2), H-Asp-Glu-NH-CH₂-CH₃ (SEQ ID NO: 3), H-Val-Asp-Glu-OH, H-Leu-Asp-Glu-OH (SEQ ID NO: 5) and H-Ala-Asp-Glu-Arg-OH (SEQ ID NO: 6), with the peptides being chemically stable upon storage.

This invention also provides a pharmaceutical composition having a cytoprotective activity and contains an effective quantity of peptides selected from a group that includes Ac-Asp-Glu-OH (SEQ ID NO: 1), H-Asp-Glu-OCH₃ (SEQ ID NO: 2), H-Asp-Glu-NH-CH₂-CH₃ (SEQ ID NO: 3), H-Val-Asp-Glu-OH (SEQ ID NO: 4), H-Leu-Asp-Glu-OH (SEQ ID NO: 5) or H-Ala-Asp-Glu-Arg-OH (SEQ ID NO: 6). The discovered cytoprotective activity makes possible the use of the proposed pharmaceutical composition in the food, cosmetological or pharmaceutical industries.

One example of a peptide acetylated at the N-terminal end of the peptide Ac-Asp-Glu-OH (AcDE) (SEQ ID NO: 1). The illustrating examples of compounds with the complex ester bound at the C-terminus are the peptides H-Asp-Glu-OCH₃ (SEQ ID NO: 2). An example of a compound with an amide bound at the C-terminal is the peptide H-Asp-Glu-NH-CH₂-CH₃ (SEQ ID NO: 3).

The peptides are synthesized with methodological techniques generally accepted in the chemistry of biologically active peptides [A.A. Gershkovich, V.K. Kibirev, Chemical synthesis of peptides. Kiev, Naukova dumka, 1992; peptides, basic method of forming peptide links, Moscow, Mir, 1983], as shown in Examples 1-6.

### Abbreviations:

Boc - tert-butyloxycarbonyl
Bzl - benzyl
Z - benzyloxycarbonyl
Bu - Tert-buty
DCHA - dicyclohexylamine
TFA - trifluoroacetic acid
ONP - paranitrophenyl
ONSu - N- oxy succinimide
NMM - N-methylmorpholine
DMF - dimethylforamide
Tos - para-toluene sulphonate
DCHA - dicyclohexylamine
Ala (A) -residue of alanine
Asp (D) - residue of aspartic acid
Glu (E) - residue of glutamic acid
Lys (K) - residue of lysine
Gly (G) - residue of glycine
Arg (R) - residue of arginine
Tip (W) - residue of tryptophan
Leu (L) - residue of leucine
DMEM - Dulbecco's Modified Eagle's Medium
FBS - fetal bovine serum
PBS - phosphate buffer saline, pH 7.4
HeLa (ATCC Number - CCL-2.2, subclone S3) - human uterus adenocarcinoma of epithelial morphology
NIH 3T3 (ATCC Number - CRL-1658) - embryonic fibroblasts of mice
DMSO - dimethylsulfoxide
EDTA - ethylene diamine tetraacetate

Stability of peptides in storage was studied in accordance with Example 7. The results obtained (Table 1) demonstrate that the peptides in accordance with the invention are stable in solutions at various pH levels.

Biological activity of the peptides was studied on the model of cell culture in mice and human: NIH 3T3 and HeLa S3, respectively (Example 8).

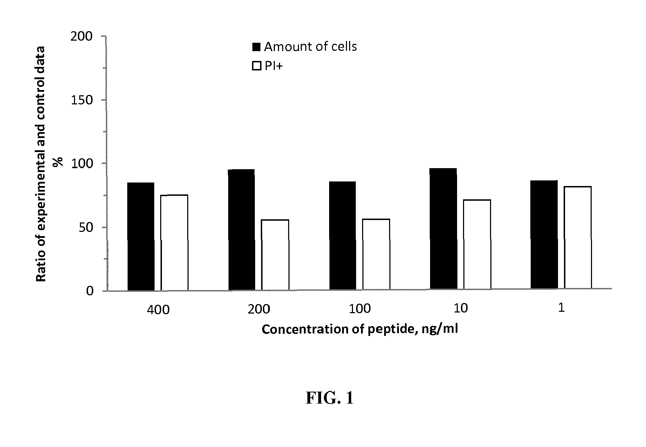

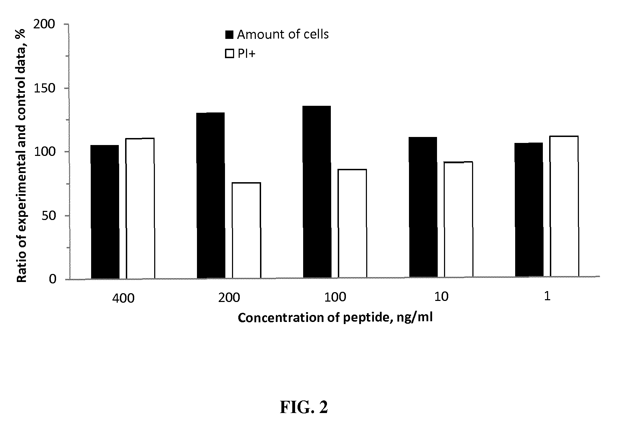

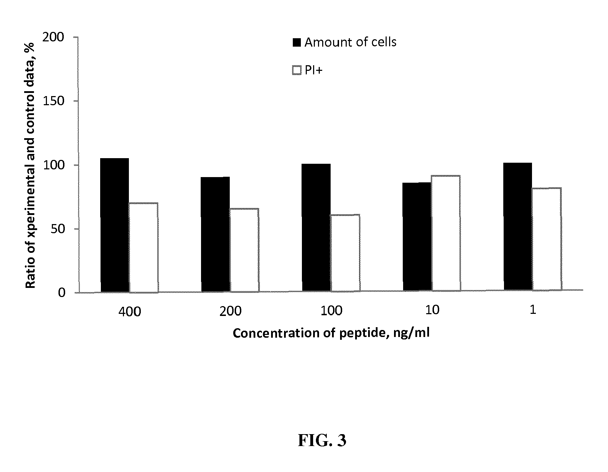

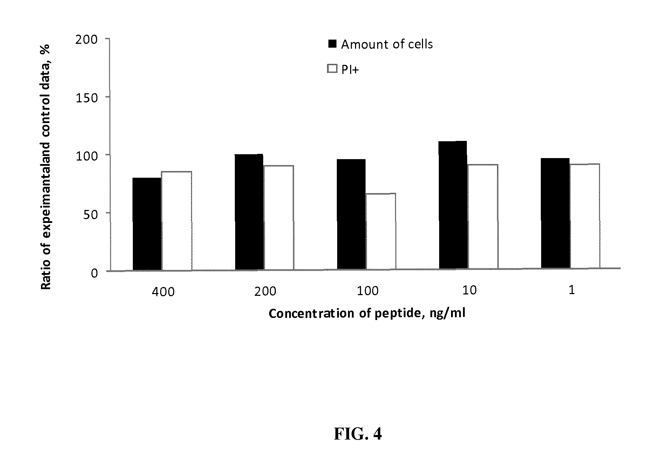

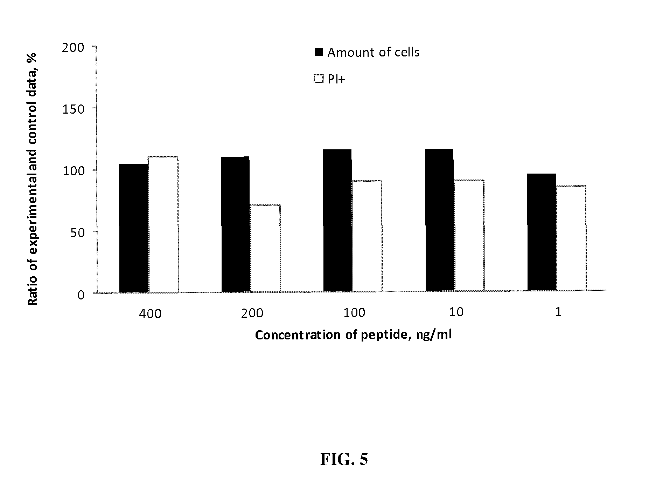

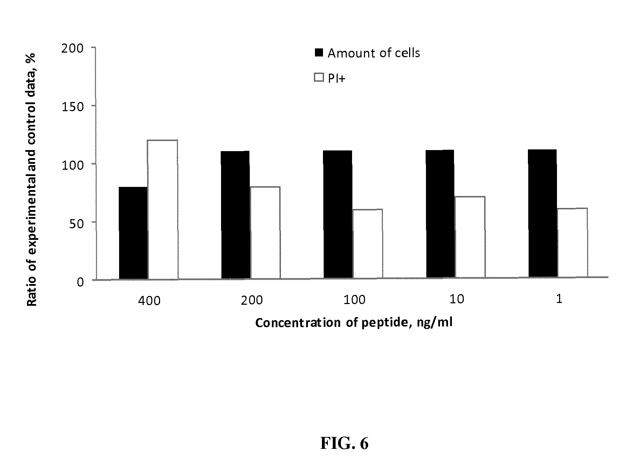

Biological activity of each peptide was determined according to the following parameters: effect on cell proliferation and ability to induce apoptosis and necrosis. For this purpose, the following experiments were performed: the ratio of NIH 3T3 cell death (with damaged plasma membrane) after processing with peptides in the total number of cells (the results are shown in **Fig. 1-6**), and the rate of apoptotic cell death.

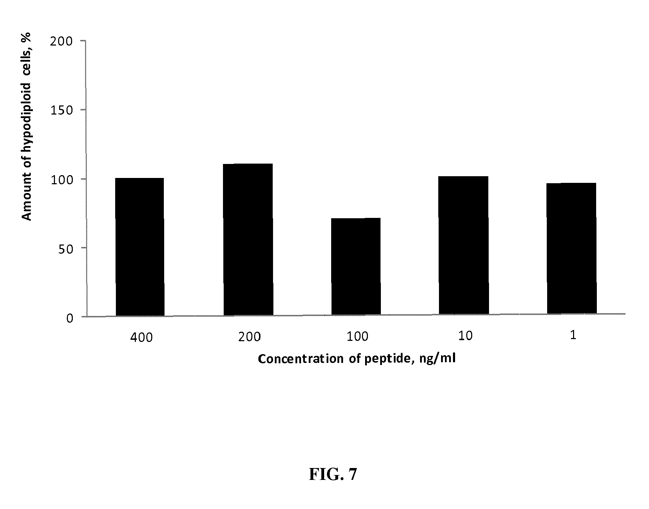

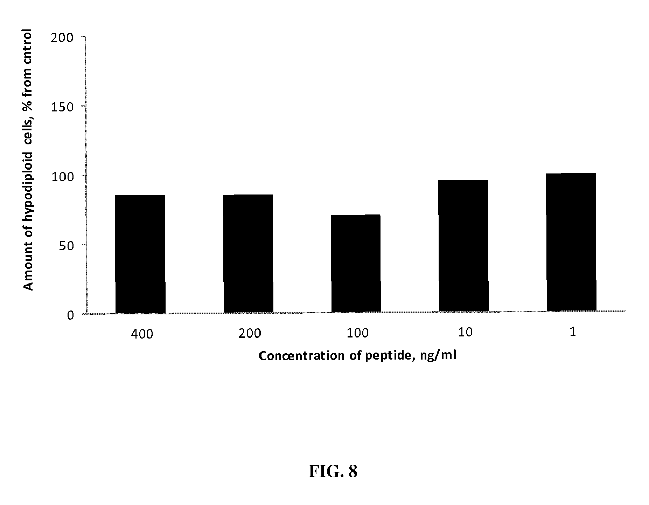

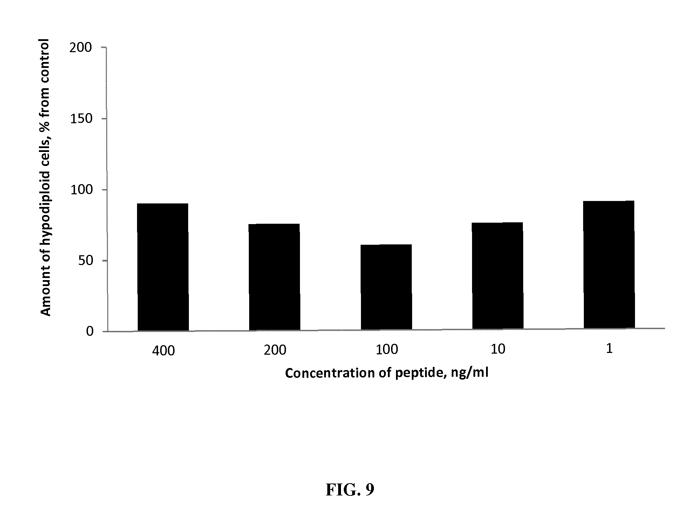

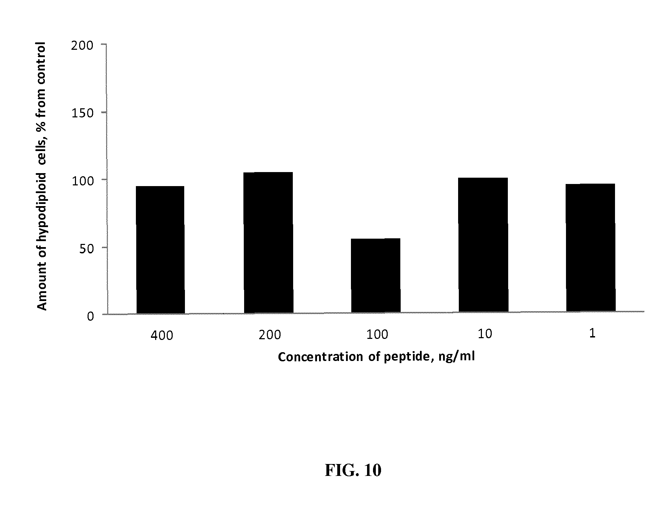

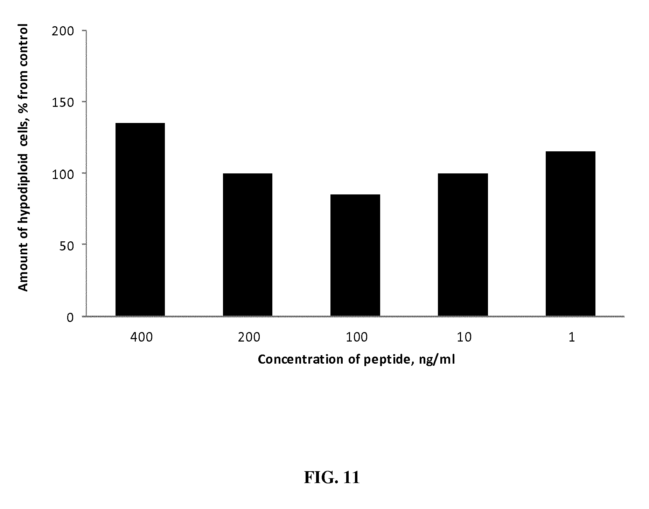

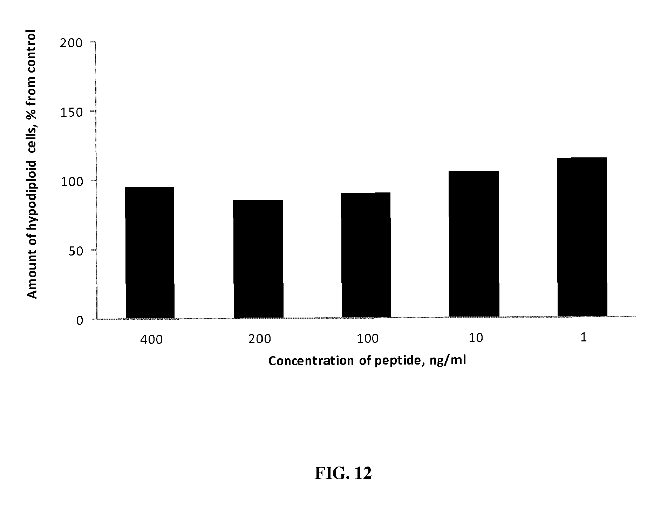

Apoptotic cell death was determined by the ratio of the HeLa S3 cells with hypodiploid content of the DNA. The method is based on the observation of fragmented DNA in cells undergoing apoptosis. This DNA is removed from the cells during processing with HCl solution. The results determining apoptotic death of the HeLa S3 cells following incubation with peptides are shown in **Fig. 7-12****.**

The protective properties of the peptides were demonstrated in experiments studying the effect of the peptides on apoptosis and necrosis in a model system of cultured mice and human cells: NIH 3T3 and HeLa S3 (Example 9).

Apoptosis refers to programmed cell death resulting from an implemented genetic program or a response to external factors, and requiring expenditure of energy and macromolecule synthesis. Apoptosis is characterized by the appearance of cytological signs (apoptosis markers) and molecular processes. Live cells demonstrate asymmetric distribution of different phospholipids between internal and external monolayers of the cytoplasmic membrane: choline-containing phospholipids such as phosphatidylcholine and sphingomyelin are localized mainly in the external monolayer, whereas phosphatidylethanolamine and phosphatidyl serine (aminophospholipids) are in the internal one. The cytoplasmic membrane undergoes changes in the process of apoptosis, including the transition of phosphatidyl serine from the internal monolayer to the external one where phosphatidyl serine becomes available for binding to Annexin-V, a calcium-dependent, phospholipid-binding protein that has a molecular mass of 35-36 kDa and a high affinity for phosphatidylcholine (K_{d} equal to 5x10⁻¹⁰ M). The Annexin-V-binding analysis is based on fast and highly specific identification of cells that contain phosphatidylcholine in the external monolayer of the cytoplasmic membrane, i.e. cells at the early stage of apoptosis.

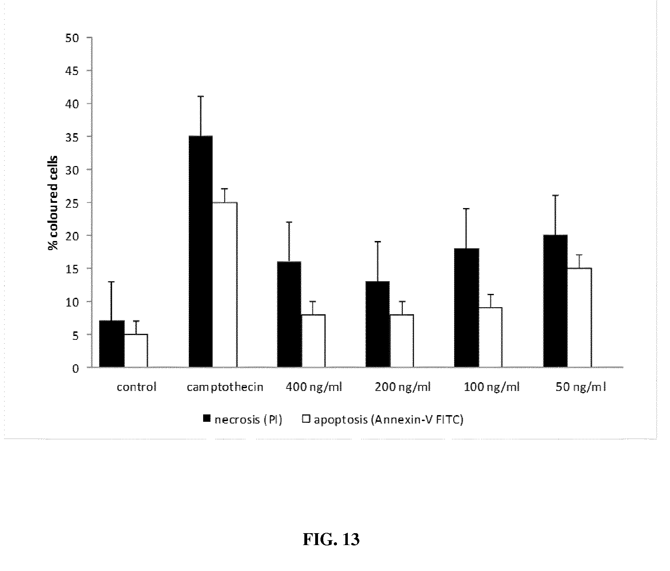

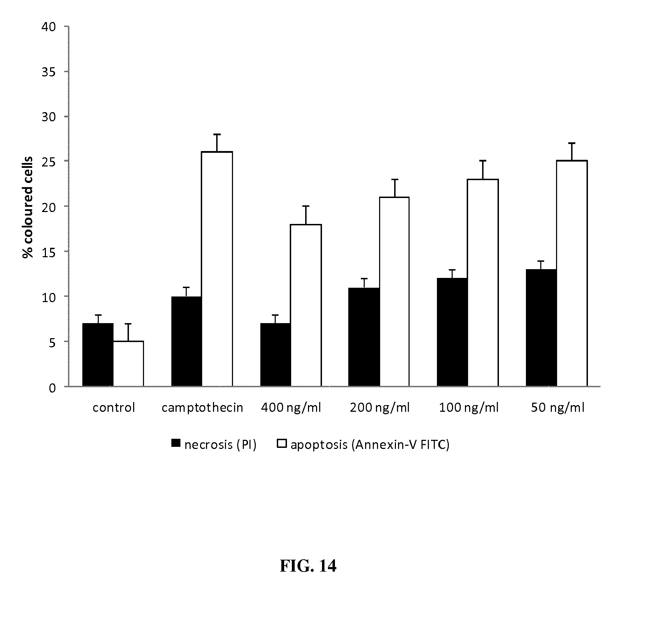

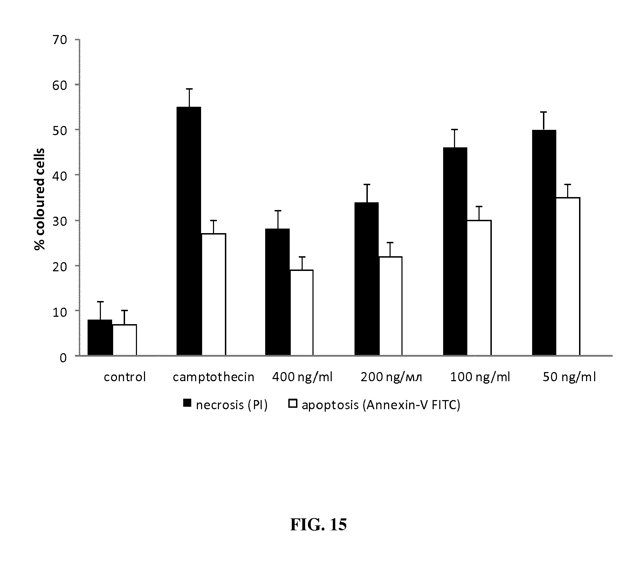

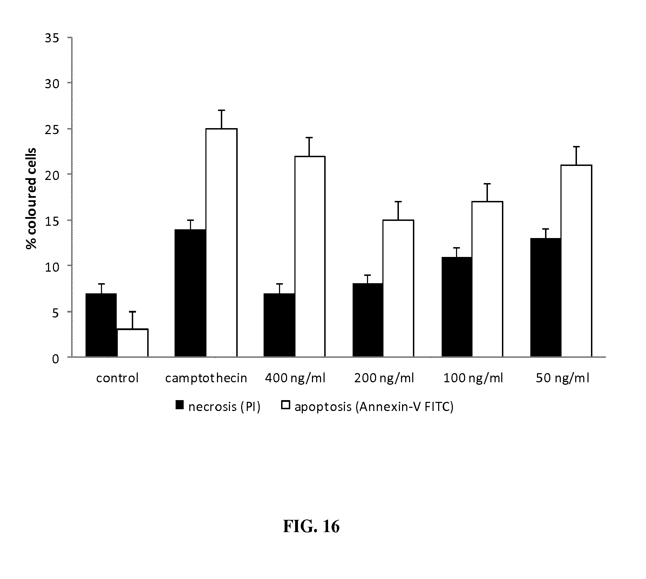

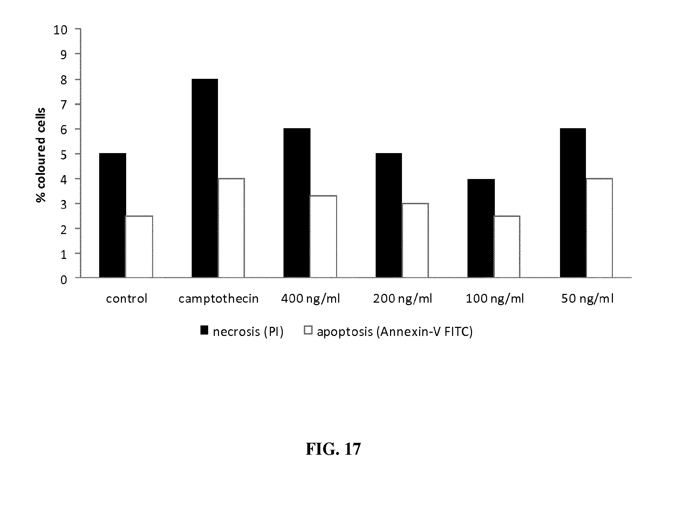

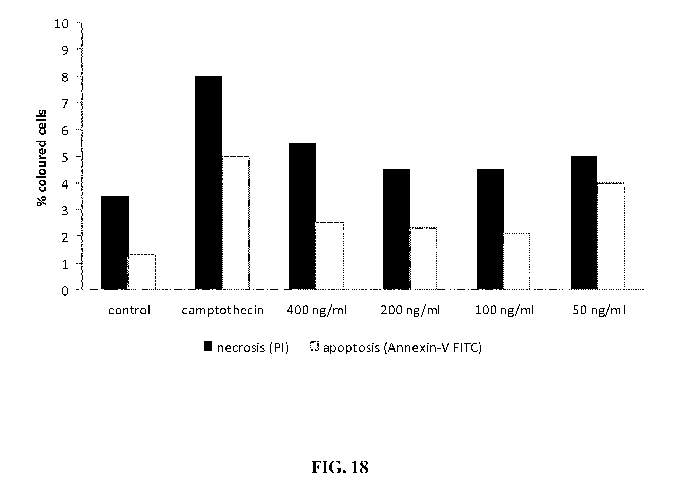

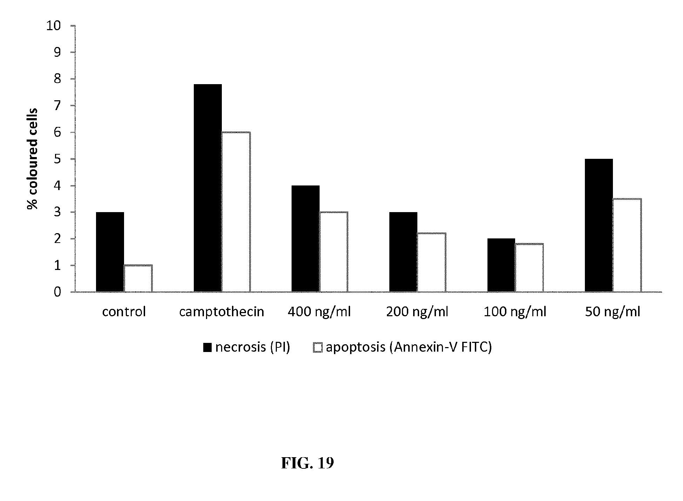

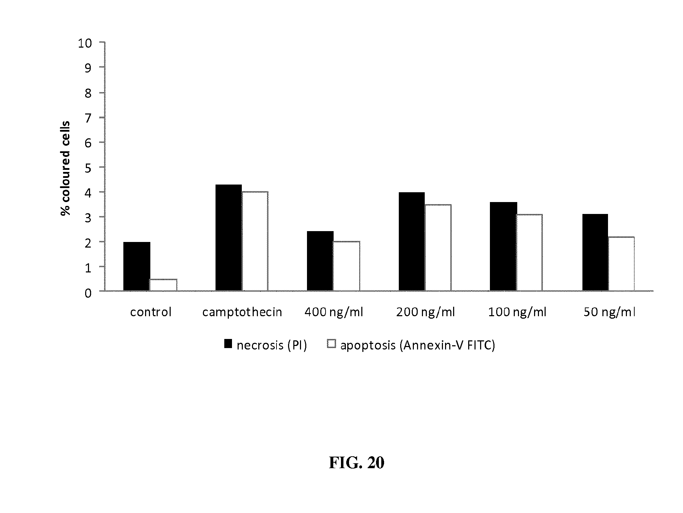

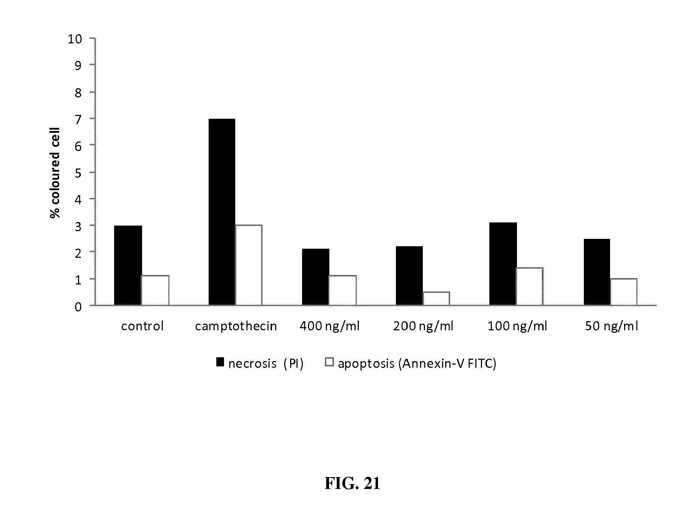

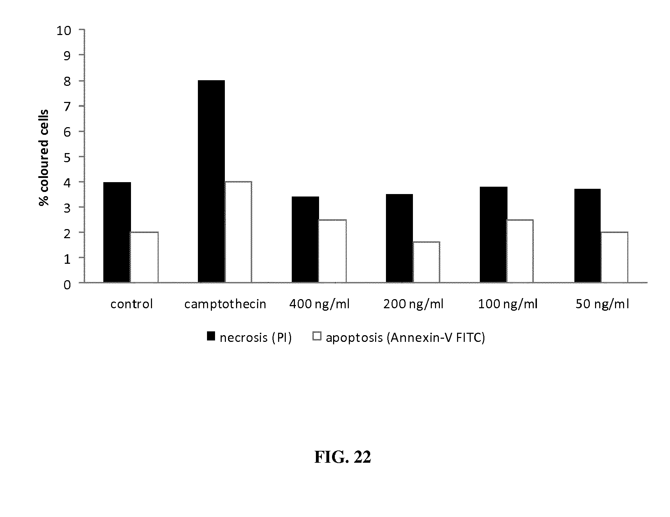

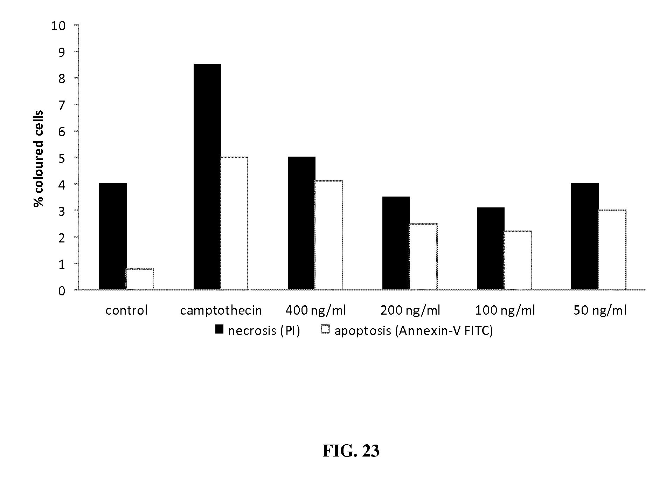

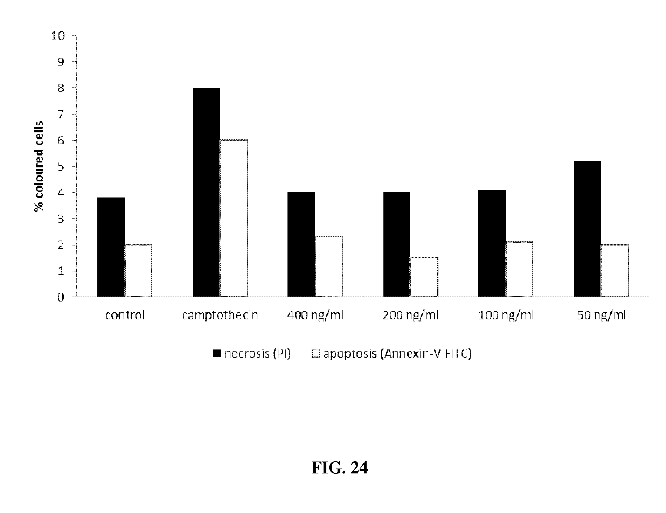

The data on the percentages of apoptotic and necrotic cells in the populations of HeLa S3 and NIH3T3 in the presence of 4 different concentrations of the peptides are shown in **Table 2**. The histograms (**Fig. 13-24**) show percentages of apoptotic and necrotic cells in the populations of HeLa S3 and NIH3T3 in the presence of 4 different concentrations of the peptides.

The histograms and data in Table 2 demonstrate that the tested peptides, in the range of concentrations from 50 to 400 ng/ml, a pronounced protective effect on the HeLa S3 and NIH3T3 cells, i.e. have a cytoprotective effect.

The pharmaceutical composition, in accordance with the invention, can be implemented in the form of tablets and capsules with the use of pharmaceutically acceptable substances and techniques traditional for this mode of administration, and can also be implemented in the form of solutions for injection. In addition, the composition can be produced in the form of drops or a solution for internal administration, or in the form of a nasal spray or nasal drops, a subglossal or oral aerosol, or a powder, or a lyophilisate for solutions.

The above forms of the pharmaceutical composition can be produced with known methods. The composition according to the invention can be administered orally or parenterally (e.g., intravenously, intramuscularly, subcutaneously). Any pharmaceutically acceptable media or solvents can be used to obtain the pharmaceutical composition according to this invention. The pharmaceutical composition for oral administration can be in the form of solutions, suspensions, tablets, pills, capsules, powders and the like. Tablets containing various excipients, e.g., sodium citrate, calcium carbonate and calcium phosphate or any other pharmaceutically acceptable excipients, are used together with various disintegrators, e.g., starch, preferably potato starch or tapioca, complex silicates or any other pharmaceutically acceptable disintegrators, together with binding agents, e.g., polyvinyl pyrrolidone, sacharose, gelatin, gum arabic and any other pharmaceutically acceptable binding agents. In addition, lubricant agents, e.g. magnesium stearate, sodium laurel sulfate, talcum for tabletization or any other pharmaceutically acceptable agents, are often used. The compositions according to the invention are also used in soft and hard filled gelatin capsules; in this case the preferred materials include, for example, lactose or sugar of milk, and also polyethyleneglycols with high molecular weight. If water suspensions and/or elixirs are desired for peroral administration, the compounds of this invention can be combined with various sweetening agents that improve taste and smell, coloring agents, emulsifiers and/or suspending substances, as well as solvents such as water, ethanol, propylene glycol, glycerin and their various combinations. Solutions in sesame seed or peanut oil or in water propylene glycol, as well as sterile water solutions of appropriate water-soluble salts, can be used for parenteral administration. Such water solutions can be buffer ones, if necessary, and a water solvent is first isotoned with a sufficient quantity of a salt or glucose solution. Such water solutions are particularly adapted for intravenous, intramuscular, subcutaneous and intraperitoneal introduction. To this end, the water media can be easily obtained with standard techniques that are well known to specialists.

The pharmaceutical composition according to the invention contains a sufficient quantity of peptides selected from the group that includes Ac-Asp-Glu-OH (SEQ ID NO: 1), H-Asp-Glu-OCH₃ (SEQ ID NO: 2), H-Asp-Glu-NH-CH2-CH₃ (SEQ ID NO: 3), H-Val-Asp-Glu-OH (SEQ ID NO: 4), H-Leu-Asp-Glu-OH (SEQ ID NO: 5) or H-Ala-Asp-Glu-Arg-OH (SEQ ID NO: 6). In its preferred embodiment, the pharmaceutical composition according to the invention contains peptide(s) with 10 to 95 weight %.

The invention is illustrated with the following examples.

### Example 1. Synthesis of the peptide Ac-Asp-Glu-OH (AcDE) (SEQ ID NO: 1).

### Boc-Asp(OBzl)-Glu (OBzl)-OBzl

16.5 ml (150 mmol) of NMM and 19.5 ml (150 mmol) of isobutylchloroformate were added to the solution of Boc-Asp (Bzl)-OH 48.5 g (150 mmol) cooled down to -20°C in 300 ml of DMF and agitated for 15 minutes. The mixture was agitated at the same temperature for 20 minutes. A solution, cooled down to -20°C, and comprising 74.5 g. (155 mmol) of the aminocomponent H-Glu (Bzl)-OBzl x Tos in 200 ml of DMF, containing 17 ml of NMM (155 mmol), was further added. The reaction mixture was agitated for 1 hour at -10°C, and for 2 hours at room temperature. Once evaporated, the residue was dissolved in ethyl acetate (500 ml) and sequentially washed with 5% NaHCO₃, H₂O, 2% H₂SO₄, H₂O (twice, with 200 ml of each), boiled down, and boiled down again with isopropyl alcohol. 500 ml of hexane and 50 ml of ester were added to the resulting oil, and the resulting oil was ground until the formation of solid particles.

The product is crystallized in a refrigerator. The sediment is filtered, washed with hexane on the filter, and dried. 89 g (89%) of a chromatographically homogeneous product were obtained.

12.64 g (20 mmol) of the protected dipeptide 1 (Asp-Glu peptide) were dissolved in 100 ml of chloroform, added to this solution at 0°C were 100 ml of trifluoroacetic acid, the mixture was kept for 1 hour at room temperature, and the solvents were evaporated in vacuum. The residue was dissolved in 300 ml of ethyl acetate and washed with 5% solution of bicarbonate 3 times, 200 ml each time. The organic layer was separated and dried over water-free Na₂SO₄, and the ethyl acetate was boiled down. The residue was dissolved in 300 ml of dimethyl formamide, added to the resulting solution were 3.6 g (20 mmol) of paranitophenyl ester of acetic acid. The mixture was left for 15 hours at room temperature. The solvent was boiled down, the residue was dissolved in ethyl acetate and washed with a 5% water solution of ammonia until traces of pararnitrophenol disappeared (control with TLC), and with water to pH 6-7; the organic layer was separated and dried, and ethyl acetate was boiled down. The residue was dissolved in 80% acetic acid and hydrated above 5% Pd\C at room temperature. Once the hydration was completed the catalyst was filtered out, the filtrate was boiled down, the residue was dissolved in 300 ml water, again boiled down to half of the volume, and lyophilized. 5.2 g (85.8%) of the target acyl derivative dipeptide were obtained.

### According to HPLC, the product's purity was over 95 %.

In the PMR spectrum:
Asp - 4.62 (α-CH); 2.70, 2.53 (β-CH2); 8.63 (NH)
Glu - 4.20 (α-CH); 1.97, 1.78 (β-CH2); 2.26(γ-CH2); 8.19 (NH)
Ac - 1.96 (3H, -COCH₃)

### Example 2. Synthesis of the peptide H-Asp-Glu-OCH₃ (SEQ ID NO: 2).

### Z-Asp(OBu^{t})-Glu(OBu^{t})-OCH₃

10.0 g (46.3 mmol) of H-Glu(OBu^{t})-OCH₃ were dissolved in 300 ml of dimethyl formamide; 19.7 g (47 mmol) of Z-Asp(OBu^{t})-ONSu were then added at room temperature. After 12 hours (control - TLC in the system chloroform:methonol: acetic acid - 9:1:0.5 « B») the reaction mixture was boiled down, the residue was dissolved in ethyl acetate, washed sequentially with 2% sulphuric acid and water, dried over Na₂SO₄ and boiled down. The residue was dissolved in 100 ml of ester. The substance crystallized at 4° C. The sediment was filtered out, washed with ester, and dried in air.

20 g (80.3 %) of chromatographically homogeneous substance were obtained.

20 g (37 mmol) of the compound obtained above were dissolved in 100 ml of chloroform, 100 ml of trifluoroacetic acid was then added to the solution. The mixture was kept for 2 hours at room temperature. The solvent was boiled down. The residue was dissolved in ethyl acetate and washed with water, a 1-2 % solution of sodium bicarbonate, and with water again. The organic layer was separated and dried, and the solvent was boiled down. The residue was dissolved in 10% water methanol and hydrated over 5% Pd/C. Upon completion of the hydration, the catalyst was filtered out, and methanol boiled down. The product was settled with ester. The sold sediment was filtered and dried in air. 9.6 g (94%) of the targeted methyl ester were obtained.

In the PMR spectrum:
Asp - 4.13 (α-CH); 2.79, 2.66 (β-CH2); 8.14 (NH2)
Glu - 4.59 (α-CH); 1.93, 1.72 (β-CH2); 2.36 (γ-CH2); 8.67 (NH).
OCH₃ - 2.8 (3H, CH₃)

### Example 3. Synthesis of the peptide H-Asp-Glu-NH - CH₂-CH₃ (DENHEt) (SEQ ID NO: 3).

### Boc-Asp(OBzl)-Glu (OBzl) -NH-CH2-CH3

2.22 ml (20 mmol) of N-methylmorpholine and 2.6 ml (20 mmol) isobutylchloroformate were added to -20°C solution of 6.46 g (20 mmol) of Boc-Asp (OBzl)-OH in 100 ml of dimethyl formamide, this was stirred for 15 minutes and a -20°C solution of 5.0 g (21 mmol) of aminocomponent H-Glu (Bzl)-NH Et were added to 40 ml of dimethyl formamide dimethyl. The reaction mixture was stirred for 1 hour at -10°C, and for 2 hours at room temperature. It was boiled down, the residue was dissolved in ethyl acetate (250 ml) and sequentially washed with a 2% solution of sulphuric acid, a 5% solution of bicarbonate, water (twice, 200 ml of each), boiled down, boiled down again with isopropyl alcohol. 50 ml of hexane and 50 ml of ester were added to the resulting oil, which was then ground until solid particles appeared. The product was crystallized in a refrigerator. The sediment was filtered out, washed on the filter with hexane and dried. 9.9 g (85%) of a chromatographically homogeneous product were obtained. 5.86 g (10 mmol) of the protected peptide, obtained as described above, were dissolved in 50 ml of chloroform; 50 ml trifluoroacetic acid at 0°C were added to the solution obtained, the mixture was kept for 1 hour at room temperature, and the solvents were evaporated in vacuum. The residue was dissolved in 100 ml of ethyl acetate and washed with a 5% solution of bicarbonate 3 times, 200 ml each time. The ethyl acetate was evaporated. The residue was dissolved in 80% acetic acid and hydrated over 5% palladium on carbon (Pd\C) at room temperature. Upon completion of hydration (control with TLC), the catalyst was filtered out, the filtrate was evaporated, and the residue was dissolved in 300 ml of water, it was again evaporated to half of the volume and lyophilized. 2.57 g (95%) of the target dipeptide were obtained. According to HPLC, the product's purity exceeded 95 %.

In the PMR spectrum:
Asp - 4.13 (α-CH); 2.79, 2.66 (β-CH2); 8.14 (NH2)
Glu - 4.59 (α-CH); 1.93, 1.72 (β -CH2); 2.36 (γ-CH2); 8.67 (NH).
NH-CH2-CH3 - 2.45 (CH2), 1.43 (CH3), 7.56 (NH)

### Example 4. Synthesis of the peptide H-Ala-Asp-Glu-Arg-OH (ADER) (SEQ ID NO: 6).

### Z-Glu(OtBu)-ArgOH

38.3 g (228 mmol) of H-Arg-OH were added to 104.4 g (228 mmol) of a Z-Glu(OtBu)-ONP solution in 0.5 1 of dimethyl formamide. The reaction mixture was stirred for 24 hours at room temperature. It was then kept at +4°C for four hours; the resulting sediment was filtered out and washed on the filter with 0.4 1 of a mixture of dimethyl formamide-ester (1:2), 0.45 1 of ester, and 0.4 1 of hexane. It was dried. Yield: 91.7 g (82.3%) of chromatographically homogeneous Z-Glu(OtBu)ArgOH, control in the chloroform-methanol-acetic acid (5:3:1) system.

### Z-Asp(OBu)-Glu(OBu)-Arg-OH

The solution of 90.6 g (185 mmol) of Z-Glu(OtBut)-ArgOH in 1.2 1 of ethanol was hydrated over 10 g of 5% Pd/C. Upon completion of the hydration (control in TLC in the system of chloroform-methanol-acetic acid, 5:3:1), the catalyst was filtered out, and the solvent was evaporated. The resulting oil product was dissolved in 500 ml of dimethyl formamide, and 75.9 g (185 mmol) of Z-Asp(OtBu)-ONP were added to the resulting solution; the reaction mixture was left at room temperature for 15 hours till the completion of the reaction (control in the system of chloroform-methanol-acetic acid, 5:3:1). The solvent was evaporated, the resulting oil was dissolved in 150 ml of chloroform and spread on a column with silica gel (400 g), washed with chloroform until removal of nitrophenol, 10% ethanol in chloroform, washed away with 30% ethanol in chloroform, and evaporated. (Control in the system: chloroform-methanol-acetic acid, 5:3:1). The fractions containing the targeted product were united, and the solvent evaporated. Added to the remainder were 400 ml of diethyl ester, the sediment was filtered, washed on the filter with ester, and dried. 103 g (84%) chromatographically homogeneous protected tripeptide were obtained.

The solution of 100 g (150 mmol) of Z-Asp(Bu)-Glu(OtBut)-ArgOH in 1.2 1 of ethanol was hydrated over 10 g of 5% Pd/C. Upon completion of the hydration (control with TLC in the system of chloroform-methanol-acetic acid, 5:3:1), the catalyst was filtered out, and the solvent was evaporated. The resulting oil product was dissolved in 500 ml of dimethyl formamide, and 51.6 g (150 mmol) of Z-Ala-ONp were added to the resulting solution. The reaction mixture was left at room temperature for 15 hours until the completion of the reaction (control in the system of chloroform-methanol-acetic acid, 5:3:1). The solvent was evaporated, the resulting oil was dissolved in 150 ml of chloroform and spread on a column with silica gel (about 400 g), washed with chloroform until removal of nitrophenol, 10% ethanol in chloroform, and washed away with 30% ethanol in chloroform, then evaporated. (Control in the system of chloroform-methanol-acetic acid, 5:3:1). The fractions with the targeted product were united, the solvents were evaporated. Added to the residue were 400 ml of diethyl ester, the sediment was filtered out, washed on the filter with ester and dried. 95 g (86%) of chromatographically homogeneous protected tetrapeptide were obtained. The product was dissolved in 500 ml of trifluoroacetic acid, and the mixture was kept for 2 hours at room temperature. The acid was evaporated, and 1000 ml of diethyl ester were added to the residue, the resulting amorphous residue was filtered, washed on the filter twice with 150 ml each time, and dried. The substance was dissolved in 1500 ml of a mixture of water with ethanol, and hydrated in the flow of hydrogen over 10% Pd/C. Upon the completion of hydration (control with TLC), the catalyst was filtered out, the solvents were evaporated to oil, dissolved in 300 ml of water and applied on a column with SP-sephadex. The column was washed with two volumes of 0.05 M pyridine-acetate buffer, and the substance was washed off with the same 0.2 M buffer. The fractions containing the product were united, the solvents were evaporated, a second evaporation with water was carried out to remove residues of pyridine acetate, and liophilization was carried out. 60 g (82%) of the target product were obtained with purity exceeding 95% according to HPLC.

In the PMR spectrum:
Ala - 3.75 (α-CH); 1.42 (β - CH3); 8.02 (2H, NH2)
Asp - 4.16 (α-CH); 2.82, 2.65 (β-CH2); 8.14 (NH)
Glu - 4.35 (α-CH) 1.93, 1.87 (β-CH2); 2.30 (γ-CH2); 8.60 (NH).
Arg - 4.16 (α-CH); 1.75,1.62 (β-CH2); 1.54,1.50 (γ-CH2); 3.10 (δ - CH2); 7.58 (ε-NH₂); 8.28 (NH).

### Example 5. Synthesis of the peptide H-Val-Asp-Glu-OH (VED) (SEQ ID NO: 4).

### Z-Val-Asp(OBzl)-Glu(OBzl)₂

10 g (15.8 mmol) of Boc-Asp(OBzl)-Glu(OBzl)₂ were dissolved in 200 ml of TFA, kept for 1 hour at room temperature; the acid was evaporated, the residue was dissolved in 500 ml of dry DMF, and portions of Na₂CO₃ were added until the cessation of the emission of CO₂. The residue was filtered, washed with 100 ml of DMF, added 3.33 ml (30 mmol) of N- methylmorpholine and 5.57 g (16 mmol) of Z-Val-ONSu, left for 15 hours at room temperature. The reaction mixture was evaporated by 80%, 800 ml of isopropyl alcohol were added to the resulting syrupy residue, held for 2 hours at +4°C, the sediment was filtered, washed with isopropyl alcohol and ester, and dried. Yield: 10.3 g (82%) of the product.

10.3 g (13.4 mmol) of the protected tripeptide, heated to +50°C, were dissolved in 1000 ml of AcOH + 100 ml of water until complete dilution of the sediment, and then hydrated, periodically heating the reaction mixture. Upon completion of the reaction (control with TLC), the catalyst was filtered, the filtrate was evaporated to the consistency of liquid butter, and 800 ml of isopropyl alcohol were added. The resulting sediment was filtered, washed with isopropyl alcohol, ester, and dried. The substance was dissolved in deionized water, the remainder of isopropyl alcohol was evaporated, and lyophilized. 4.4 g (92 %) of the target product with purity above 95%, as determined by HPLC, were obtained.

In the PMR spectrum:
Val - 0.94, 0.97 (γ-CH₃); 418 (α-CH); 2.13 (β-CH); 8.08 (NH2)
Asp - 4.62 (α-CH); 2.70, 2.53 (β-CH2); 8.63 (NH)
Glu - 4.20 (α-CH); 1.97, 1.78 (β-CH2); 2.26 (γ-CH2); 8.19 (NH)

### Example 6. Synthesis of the peptide H-Leu-Asp-Glu-OH (LDE) (SEQ ID NO: 5).

### Z-Leu-Asp(OBzl)-Glu(OBzl)₂

12.6 g (20.0 mmol) of Boc-Asp(OBzl)-Glu(OBzl)₂ were dissolved in 200 ml of TFA, held for 1 hour at room temperature; the acid was boiled down, the remainder was dissolved in 500 ml of dry DMF, adding portions of Na₂CO₃ until the discharge of CO₂ stopped. The sediment was filtered away, washed with 100 ml of DMF, 3.33 ml (30 mmol) of N-methylmorpholine and 5.0 g (20 mmol) of Z-Leu-ONSu were added, and left for 15 hours for room temperature. The reaction mixture was evaporated by 80%, adding to the resulting syrupy remainder 800 ml of isopropyl alcohol, and kept for 2 hours at +4°C; the resulting sediment was filtered, washed with isopropyl alcohol, ester, and dried. Yield: 13.3 g (84%) of the product.

13g (16.7 mmol) of the protected tripeptide heated to +50°C were dissolved in 1000 ml of AcOH + 100 ml of water until the complete dissolution of the sediment, and then hydrated, periodically heating the reaction mixture. Upon completion of the reaction (control with TLC), the catalyst was filtered, the filtrate was evaporated to the consistency of liquid butter, and 800 ml of isopropyl alcohol were added. The resulting sediment was filtered, washed with isopropyl alcohol, ester, and dried. The substance was dissolved in deionized water, the remainder of isopropyl alcohol was evaporated, and lyophilized. 5.5 g (87 %) of the target product with purity over 95% according to HPLC were obtained.

In the PMR spectrum:
Leu - 0.94, 0.90 (δ-CH₃); 4,38 α-CH); 1.92 (β-CH2); 1.65 (γ-CH); 8.08 (NH2)
Asp - 4.62 (α-CH); 2.70, 2.53 (β-CH2); 8.63 (NH)
Glu - 4.20 (α-CH); 1.97, 1.78 (β-CH2); 2.26 (γ-CH2); 8.19 (NH)

### Example 7. Study of stability of peptides in storage.

The peptides were dissolved in 0.05 M of an ammonium acetate buffer with pH 4.5 and pH 7.5 so that their concentrations achieved approximately 1 mg/ml. The solutions were sterilely filtered and kept in sterile, well-sealed vials. Upon expiration of the storage period the substances were analyzed with HPLC. The results are presented in **Table 1**.

**Table 1. Peptide stability in storage.**

| Peptide | Duration (time, days) | | | | | | | | | |
|---|---|---|---|---|---|---|---|---|---|---|
| | 1hour | | 10 | | 30 | | 60 | | 90 | |
| | Conditions of storage (pH, 22°C) | | | | | | | | | |
| | 4.5 | 7.5 | 4.5 | 7.5 | 4.5 | 7.5 | 4.5 | 7.5 | 4.5 | 7.5 |
| DENHEt | 98.1 | 98.2 | 98.3 | 98.2 | 98.15 | 98.10 | 98.0 | 98.0 | 97.9 | 97.85 |
| AcDE | 98.0 | 98.2 | 98.2 | 98.1 | 98.1 | 98.1 | 98.0 | 98.05 | 97.8 | 97.7 |
| VDE | 98.1 | 98.1 | 98.0 | 97.7 | 97.6 | 97.6 | 97.55 | 97.55 | 97.55 | 97.2 |
| LDE | 96.5 | 96.5 | 96.4 | 96.4 | 96.4 | 96.4 | 96.2 | 96.2 | 96.2 | 95.6 |
| ADER | 98.1 | 98.1 | 98.0 | 98.0 | 97.9 | 97.7 | 98.0 | 97.7 | 97.8 | 97.6 |
| DEOMe | 98.0 | 98.0 | 98.1 | 98.0 | 97.7 | 97.65 | 97.55 | 97.5 | 97.5 | 97.2 |

The results of analysis show that the products keep well in solutions with different values of pH.

### Example 8. Study of biological activity of the peptides in standard cultivation of cells

Biological activity of the synthesized peptides in five different concentrations (400, 200, 100, 10 and 1 ng/ml) was studied in a model of cultured mice and human cells: NIH 3T3 and HeLa S3.

Biological activity of each peptide was determined in accordance with the following parameters: the peptides' influence on the proliferation activity of cells, and the capacity of the peptides to cause apoptosis and necrosis. The study was concerned with the effect of the peptides on fibroblasts NIH 3T3 (determination of the percentage of dead cells after incubation with the peptides, and the total number of cells), and the rate of apoptotic deaths of the HeLa S3 cells after incubation with the peptides.

### Culture of cells and processing of peptides.

Fibroblasts NIH 3T3 were cultivated in the growth medium DMEM (Paneko, RF), containing 10 % of embryonic serum (Paneko), 2 U/ml of gentamicin (Paneko), glutamine 29.2 µg/ml (Paneko), in a CO₂-incubator. Culture was done using vials of SPL (South Korea) with an area of 25 cm² and 175 cm². Cell passage was done in the ratio of 1:5 - 1:7. Once approximately 50 % confluence was achieved, the peptides in the above concentrations were added into the vials. Each experiment used two vials with the same concentration of each peptide. Corresponding volume of the medium without peptides was added into control vials. Each peptide was subjected to two independent experiments (with two repetitions in each). After 24 hours of incubation with the peptides the cells were removed from the culture vial by processing them in a mixture of trypsin and versene (1:3, Paneko). The cells from each vial were placed in the same volume of buffered physiological solution (0.01 M PBS, pH 7.2). The cell suspension was divided into three equal parts in order to study the indicators mentioned above. The HeLa cells were cultivated in a similar manner, except that the cell passage was in the ratio of 1:3-1:4.

The indicators were studied using flow cytometry. A flow cytofluorimeter-sorter FACS Vantage (Becton Dickinson, USA) was used. Prior to each series of analysis, the device was tested and calibrated using standard microspheres (Polyscience), in accordance with the methodology recommended by the manufacturer.

### Determination of the total number of cells in a vial, and the percentage of dead cells

Added to the cell suspension obtained as described above were propidium iodide (propidium iodide -PI, Sigma, CIIIA) in the final concentration of 10 µg/ml, and calibration beads Calibrite (Becton Dickinson, USA) marked with fluoresceineisothiocyanates (FITC). The final concentration of calibration beads was determined with the help of a Goryaev chamber. Following incubation for 10-12 minutes, they were immediately analyzed in the flow cytometer according to four parameters: forward scatter, side scatter, PI fluorescence, and FITC fluorescence. 20 thousand cells were analyzed in each sample. The data were recorded in a file. The data were then processed using CellQuestPro (Becton Dickinson, USA).

The percentage of PI+cells with damaged plasmatic membrane (dead cells) was determined. The death rate in the control vials (15-20 units) was averaged and taken as 100%. The peptides' possible effect was assessed against the control.

FITC+ calibration beads were identified in each file, and their number was determined. Light scatter indicators were then used to identify cells, and their number was found in the same files. The ratio of cells/calibration beads was calculated in each file (sample) and used to assess the number of cells in a vial. The cells/calibration beads ratio in the control was taken as 100 %. The number of cells in the experimental vial was assessed in percentages against the control.

The results are presented in **Fig. 1-6****.**

### Determination of death of cells through apoptosis

The method is based on the fact that fragmented DNA appears in cells subjected to apoptotic death. This DNA is removed from the cells during processing with an HCl solution. The DNA remaining in the cells is stained with specific dyes, for example PI (propidium iodide), and the content of DNA is determined in cells. The cell apoptosis is determined based on the hypodiploid DNA content.

The cell suspension, obtained as described above, was cooled to +4°C. Added to the cell suspension was cooled ethanol in the volume ratio of 1:3, with constant agitation. The cells were stored for 1-2 weeks prior to analysis. Ethanol was centrifuged from the samples, the fragmented DNA was then removed from the cells with 0.6M HCl (37°C, 10 minutes.). The cells were then washed with PBS and stained in 0.3 ml of a PI solution (50 µg/ml) in PBS, kept for 15 minutes at room temperature and analyzed in the flow cytometer. At this concentration, PI makes it possible to determine the DNA content cell by cell and to identify the cell fraction with the fragmented DNA at high resolution. The PI fluorescence was determined with narrow-band filters of 585/42 nm, with the laser power equal to 57 (milliwatt or mWt). 5·10³ cells were analyzed in each sample, this was followed by computer processing using CellQuestPro (Becton Dickinson, USA). At the first stage of the analysis, a region of cells was identified with light scatter, then the PI fluorescence was assessed in the region. The DNA hypodiploid content marker was set using the control samples.

The results of determination of the HeLa S3 apoptosis after incubation with the peptides are shown in **Fig. 7-12****.**

### Example 9. Study of protective properties of peptides.

The experiments to study the effect of peptides on apoptosis and necrosis involved the use of reagents in the ApoTarget Annexin-V FITC Apoptosis Kit (Invitrogen, CIIIA). In addition to Annexin-V, marked by the FITC fluorescent stain, the ApoTarget Annexin-V FITC Apoptosis Kit contains propidium iodide (PI), a dye. PI can permeate only those cells whose cytoplasmic membrane is damaged, which is a characteristic sign of an advanced stage of apoptosis or necrosis. Having permeated cells with damaged cell membrane, PI binds itself to the DNA. The «red» fluorescence of resulting complexes is identified with a flow cytofluorimeter.

Therefore, the procedure of double staining (Annexin-V FITC plus PI) and the process of flow cytofluorimetry can be used to distinguish 3 cell populations and determine their percentage relationship: 1) non-apoptotic cells (Annexin-V-negative and PI-negative), 2) cells at an early stage of apoptosis (Annexin-V-positive and PI-negative), 3) necrotic cells or cells at an advanced stage of apoptosis (Annexin-V- positive and PI- positive). An increase in the percentage of apoptotic and necrotic cells can serve as a measure of how the compounds added to the culture medium act on these processes. Camptothecin (Camptothecin, Sigma, CIIIA), which is recommended by the manufacturer, was used as control in the experiments because of its pronounced ability to induce apoptosis and necrosis of cells, especially tumor ones.

Peptide solutions in sterile PBS with concentrations of 1 mg/ml were prepared immediately before the experiments to study the peptides' effect on apoptosis and necrosis. The same solutions that were stored in sterile test tubes at +4°C were used to study the proliferation activity. Working dilutions of peptides in the cell cultivation medium were prepared on the day of each experiment. A sterile plastic 96-well plate (Corning, USA) was used for this purpose. The dilutions were prepared so that the introduction of 25 µl of each working dilution in an experimental well with cultivated cells would result in final concentrations of peptides equal to 400, 200, 100 and 50 ng/ml.

### Culture of cells

The HeLa S3 and NIH 3T3 cells were cultivated with the use of reagents manufactured by Invitrogen (USA) and plastic vials, plates, pipettes and filters of Corning (USA). The cells were cultivated in DMEM, prepared with a dry concentrate dissolved in pyrogen-free distilled water (Millipore, USA). The medium contained 10 % FBS, 2 mM of glutamine and penicillin-streptomycin (diluted according to the vendor's recommendations). HeLa S3 and NIH 3T3 were grown in vials T150 at +37 °C in the atmosphere of 5.6 % CO₂. The cells were constantly maintained in the logarithmic growth phase, to this end they were passed at least 2 times a week, with trypsin used to remove cells from the walls of the culture vials. Live cells were counted in a hemacytometer after staining the suspension aliquot with trypan blue.

The HeLa S3 and NIH 3T3 cells were removed from the surface of the culture vials and counted; the concentration was moved to 10⁵ cell/ml, and the resulting suspensions were put in doses of 1 ml in the wells of a 24-well plate.

Having made sure on the following day that the cells adhered to the plate's surface, the culture medium was replaced with a fresh one in each well.

Camptothecin was added in the control wells (positive control - stimulation of apoptosis and necrosis) to the final concentration of 50 µM that had been determined in preliminary experiments with the NIH 3T3 and HeLa S3 cells. 25 µl of fresh culture medium were added in the control wells (negative control), 25 µl of working peptide dilutions were added in each experimental well (each working dilution - twice), as well as camptothecin in the same final concentration. Incubation was conducted at +37 °C.

24 hours after the beginning of the incubation, the cells were removed from the surface of the 24-well plates; they were transferred from each well into a separate centrifuge test-tube of 1.5 ml, washed with 1 ml of the working buffer solution to bind the Annexin-V FITC dye, and the cells were sedimented by centrifuging (Sysmex Platelet Centrifuge PC-810). The supernatant was drained off, 5 µl of Annexin-V FITC and 10 µl of propidium iodide (PI) were added to the cell sediment that was incubated for 15 minutes in the dark at room temperature (+25 °C). 300 µl of the working buffer solution were added in each test-tube to bind Annexin-V FITC, and the intensity of green and red fluorescence was measured.

Two populations were distinguished in the distributions obtained - Annexin-V-positive and PI-negative (cells at the apoptosis stage) and Annexin-V-positive and PI-positive cells (cells at the necrosis stage).

The histograms (**Fig. 13-24**) and **Table 2** show percentages of apoptotic and necrotic cells in the HeLa S3 and NIH3T3 populations in the presence of the peptides in 4 different concentrations.

**Table 2. Percentage relationship of apoptotic and necrotic cells in the populations of HeLa S3 and NIH3T3 in the presence of peptides in 4 different concentrations.**

| Effect of peptide H-Asp-Glu-NH-CH₂-CH₃ (DENHEt) (SEQ ID NO: 3) in HeLa cell culture | | | | | | |
|---|---|---|---|---|---|---|
| | control | camptothecin | 400 ng/ml | 200 ng/ml | 100 ng/ml | 50 ng/ml |
| Necrosis | 7 | 35 | 16 | 13 | 18 | 20 |
| Apoptosis | 5 | 25 | 8 | 8 | 9 | 15 |

| Effect of peptide DENHEt (SEQ ID NO: 3) in NIH3T3 cell culture | | | | | | |
|---|---|---|---|---|---|---|
| | control | camptothecin | 400 ng/ml | 200 ng/ml | 100 ng/ml | 50 ng/ml |
| Necrosis | 7 | 10 | 7 | 11 | 12 | 13 |
| Apoptosis | 5 | 26 | 18 | 21 | 23 | 25 |

| Effect of peptide Ac-Asp-Glu-OH (AcDE) (SEQ ID NO: 1) in HeLa cell culture | | | | | | |
|---|---|---|---|---|---|---|
| | control | camptothecin | 400 ng/ml | 200 ng/ml | 100 ng/ml | 50 ng/ml |
| Necrosis | 7 | 14 | 7 | 8 | 11 | 13 |
| Apoptosis | 3 | 25 | 22 | 15 | 17 | 21 |

| Effect of peptide AcDE (SEQ ID NO: 1) in NIH3T3 cell culture | | | | | | |
|---|---|---|---|---|---|---|
| | control | camptothecin | 400 ng/ml | 200 ng/ml | 100 ng/ml | 50 ng/ml |
| Necrosis | 8 | 55 | 28 | 34 | 46 | 50 |
| Apoptosis | 7 | 27 | 19 | 22 | 30 | 35 |

| Effect of peptide H-Val-Asp-Glu-OH (VDE) (SEQ ID NO: 4) in HeLa cell culture | | | | | | |
|---|---|---|---|---|---|---|
| | control | camptothecin | 400 ng/ml | 200 ng/ml | 100 ng/ml | 50 ng/ml |
| Necrosis | 2 | 4.3 | 2.4 | 4 | 3.6 | 3.1 |
| Apoptosis | 0.5 | 4 | 2 | 3.5 | 3.1 | 2.2 |

| Effect of peptide VDE (SEQ ID NO: 4) in NIH3T3 cell culture | | | | | | |
|---|---|---|---|---|---|---|
| | control | camptothecin | 400 ng/ml | 200 ng/ml | 100 ng/ml | 50 ng/ml |
| Necrosis | 3 | 7.8 | 4 | 3 | 2 | 5 |
| Apoptosis | 1 | 6 | 3 | 2.2 | 1.8 | 3.5 |

| Effect of peptide H-Leu-Asp- Glu-OH (LDE) (SEQ ID NO: 5) in HeLa cell culture | | | | | | |
|---|---|---|---|---|---|---|
| | control | camptothecin | 400 ng/ml | 200 ng/ml | 100 ng/ml | 50 ng/ml |
| Necrosis | 3 | 7 | 2.1 | 2.2 | 3.1 | 2.5 |
| Apoptosis | 1.1 | 3 | 1 | 0.5 | 1.4 | 1 |

| Effect of peptide LDE (SEQ ID NO: 5) in NIH3T3 cell culture | | | | | | |
|---|---|---|---|---|---|---|
| | control | camptothecin | 400 ng/ml | 200 ng/ml | 100 ng/ml | 50 ng/ml |
| Necrosis | 4 | 8 | 3.4 | 3.5 | 3.8 | 3.7 |
| Apoptosis | 2 | 4 | 2.5 | 1.6 | 2.5 | 2 |

| Effect of peptide H-Ala-Asp-Glu-Arg-OH (ADER) (SEQ ID NO: 6) in HeLa cell culture | | | | | | |
|---|---|---|---|---|---|---|
| | control | camptothecin | 400 ng/ml | 200 ng/ml | 100 ng/ml | 50 ng/ml |
| Necrosis | 3.8 | 8 | 4 | 4 | 4.1 | 5.2 |
| Apoptosis | 2 | 6 | 2.3 | 1.5 | 2.1 | 2 |

| Effect of peptide ADER (SEQ ID NO: 6) in NIH3T3 cell culture | | | | | | |
|---|---|---|---|---|---|---|
| | control | camptothecin | 400 ng/ml | 200 ng/ml | 100 ng/ml | 50 ng/ml |
| Necrosis | 4 | 8.5 | 5 | 3.5 | 3.1 | 4 |
| Apoptosis | 0.8 | 5 | 4.1 | 2.5 | 2.2 | 3 |

| Effect of peptide H-Asp-Glu-OCH3 (DEOMe) (SEQ ID NO: 2) in HeLa cell culture | | | | | | |
|---|---|---|---|---|---|---|
| | control | camptothecin | 400 ng/ml | 200 ng/ml | 100 ng/ml | 50 ng/ml |
| Necrosis | 3.5 | 8 | 5.5 | 4.5 | 4.5 | 5 |
| Apoptosis | 1.3 | 5 | 2.5 | 2.3 | 2.1 | 4 |

| Effect of peptide DEOMe (SEQ ID NO: 2) in NIH3T3 cell culture | | | | | | |
|---|---|---|---|---|---|---|
| | control | camptothecin | 400 ng/ml | 200 ng/ml | 100 ng/ml | 50 ng/ml |
| Necrosis | 5 | 8 | 6 | 5 | 4 | 6 |
| Apoptosis | 2.5 | 4 | 3.3 | 3 | 2.5 | 4 |

The histograms and the data in **Table 2** show that the peptides under study, in the range of concentrations from 50 to 400 ng/ml, produce a pronounced protective effect on the HeLa S3 and NIH3T3 cells, i.e. have cytoprotective activity.

The results of the study demonstrate therefore that the medicinal agent proposed according to this invention has a pronounced cytoprotective effect and can be used in the food, cosmetological and pharmaceutical industries.

### Example 10. Preparation of forms of the composition.

### Preparation of solution for injection.

Solutions for injection were produced with a commonly known method (Chueshov V.I. et al. Industrial technology of medicines. Vo. 2, MTK-Kniga; NFAU Publishers, 2002, pp. 510-543). 180 g of a peptide substance is dissolved, while agitating and bubbling with nitrogen in 0.8 1 of water for injection, then adding water for injection to the total volume of 1.0 1. The resulting solution is subjected to sterilizing filtration in aseptic conditions, it is then poured in nitrogen into 2 ml ampoules - 1 ml per ampoule. The filled ampoules are sealed in nitrogen.

### Preparation of capsules.

Capsules were produced with a commonly known method (Chueshov V.I. et al. Industrial technology of medicines. Vo. 2, MTK-Kniga; NFAU Publishers, 2002, pp. 400-413). Ingredients are mixed based on the composition for one capsule:

### peptide - 200 mg;

excipients (monocrystaline cellulose, calcium stearate) - in quantity sufficient to obtain the content of 300 mg per capsule.

### Composition of a capsule: dyes, gelatin.

### Preparation of tablets.

Tablets were produced with a commonly known method (Chueshov V.I. et al. Industrial technology of medicines. Vo. 2, MTK-Kniga; NFAU Publishers, 2002, pp. 335-368.). Ingredients are mixed based on the composition for one tablet:
peptide - 250 mg;
colloidal silicon dioxide - 2.5 mg;
talcum - 5mg;
lactose monohydrate - 90 mg;
corn starch - 102.5 mg.

## Claims

1. Peptides having cytoprotective activity selected from a group that includes Ac-Asp-Glu-OH (SEQ ID NO: 1), H-Asp-Glu-OCH₃ (SEQ ID NO: 2), H-Asp-Glu-NH - CH₂-CH₃ (SEQ ID NO: 3), H-Val-Asp-Glu-OH (SEQ ID NO: 4), H-Leu-Asp-Glu-OH (SEQ ID NO: 5), H-Ala-Asp-Glu-Arg-OH (SEQ ID NO: 6), or their pharmaceutically acceptable salts.

2. Pharmaceutical composition having cytoprotective effect and contains effective quantities of peptides according to claim **1**.


SEQUENCE LISTING

<110> ZAMERTON HOLDINGS LIMITED
      OVCHINNIKOV, Mihail Vladimirovich

<120> PEPTIDES WITH CYTOPROTECTIVE ACTIVITY

<130> CV - 526/EP

<160> 6

<170> BiSSAP 1.0

<210> 1
<211> 2
<212> PRT
<213> Artificial Sequence

<220>
<221> SOURCE
<222> 1..2
<223> /mol_type="protein"
      /note="Peptide   -Asp-Glu-OH modified by N-terminal acetylation"
      /organism="Artificial Sequence"

<220>
<221> MOD_RES
<222> 1
<223> ACETYLATION

<400> 1
Asp Glu
1

<210> 2
<211> 2
<212> PRT
<213> Artificial Sequence

<220>
<221> SOURCE
<222> 1..2
<223> /mol_type="protein"
      /note="Peptide H-Asp-Glu-OCH3 comprising a C-terminal ester"
      /organism="Artificial Sequence"

<220>
<221> MOD_RES
<222> 2
<223> ESTER

<400> 2
Asp Glu
1

<210> 3
<211> 2
<212> PRT
<213> Artificial Sequence

<220>
<221> SOURCE
<222> 1..2

<223> /mol_type="protein"
      /note="Peptide  -Asp-Glu-NH CH2-CH3 modified by C-terminal amidat
      ion"
      /organism="Artificial Sequence"

<220>
<221> MOD_RES
<222> 2
<223> AMIDATION

<400> 3
Asp Glu
1

<210> 4
<211> 3
<212> PRT
<213> Artificial Sequence

<220>
<221> SOURCE
<222> 1..3
<223> /mol_type="protein"
      /note="Peptide H-Val-Asp-Glu-OH"
      /organism="Artificial Sequence"

<400> 4
Val Asp Glu
1

<210> 5
<211> 3
<212> PRT
<213> Artificial Sequence

<220>
<221> SOURCE
<222> 1..3
<223> /mol_type="protein"
      /note="Peptide H-Leu-Asp-Glu-OH"
      /organism="Artificial Sequence"

<400> 5
Leu Asp Glu
1

<210> 6
<211> 4
<212> PRT
<213> Artificial Sequence

<220>
<221> SOURCE
<222> 1..4
<223> /mol_type="protein"
      /note="Peptide H-Ala-Asp-Glu-Arg-OH"
      /organism="Artificial Sequence"

<400> 6
Ala Asp Glu Arg
1